# EUROPÄISCHE PATENTSCHRIFT

(11) **EP 1 385 478 B1**
(45) Veröffentlichungstag und Bekanntmachung des Hinweises auf die Patenterteilung: **15.10.2008**
(21) Anmeldenummer: 02732613.1
(22) Anmeldetag: 15.04.2002
(51) Int. Cl.: A61K 9/12, A61K 9/107, A61K 9/127

(54) **VERFAHREN ZUM AUFBRINGEN EINES WIRKSTOFFS AUF EIN SUBSTRAT, ZUSAMMENSETZUNGEN FÜR DAS VERFAHREN, BEHÄLTER ENTHALTEND EINE ZUSAMMENSETZUNG UND VERWENDUNG DER ZUSAMMENSETZUNGEN**
METHOD FOR APPLYING AN ACTIVE INGREDIENT TO A SUBSTRATE, COMPOSITIONS FOR SAID METHOD, CONTAINER CONTAINING A COMPOSITION AND USE OF SAID COMPOSITIONS
PROCEDE POUR DEPOSER UN PRINCIPE ACTIF SUR UN SUBSTRAT, COMPOSITIONS POUR CE PROCEDE, CONTENANT RECEVANT UNE TELLE COMPOSITION ET UTILISATION DE CES COMPOSITIONS

(30) Priorität: 08.05.2001 EP 01111041
(43) Veröffentlichungstag der Anmeldung: 04.02.2004
(60) Teilanmeldung: 08103821.8
(73) Patentinhaber: VESIFACT AG, 6342 Baar 2 (CH)
(72) Erfinder: SUPERSAXO, Andreas, Weder, 6340 Baar (CH); WEDER, Marc, Antoine, CH-8803 Rüschlikon (CH); WEDER, Hans, Georg, CH-8803 Rüschlikon (CH)
(74) Vertreter: Welch, Andreas
(86) Internationale Anmeldenummer: PCT/EP2002/004146
(87) Internationale Veröffentlichungsnummer: WO 2002/089769

(56) Entgegenhaltungen:
- EP-A- 0 382 619
- EP-A- 0 488 089
- EP-A- 0 626 167
- WO-A-01/28520
- WO-A-97/21428
- DE-A- 19 859 427
- KIBBE ET AL. (ED.): "Handbook of Pharmaceutical Excipients, 3rd Ed." 2000, APHA/PHP , ISBN 0-85369-381-1 * Seite 236 - Seite 237 *

## Beschreibung

Die Erfindung betrifft ein Verfahren zum Aufbringen eines Wirkstoffs auf ein Substrat, Zusammensetzungen zur Verwendung bei einem solchen Verfahren, ein Behälter zur Aufnahme der Zusammensetzung und Verwendung der Zusammensetzungen gemäss Oberbegriff der unabhängigen Patentansprüche.

Der Applikationsform von Wirkstoffen kommt heute eine besondere Bedeutung zu. Die Art und Weise wie ein Wirkstoff angeboten wird, entscheidet darüber, welche Menge eines Wirkstoffs in welcher Zeitspanne von einem Applikationsort an den Wirkungsort gelangt und damit auch welche Menge an Wirkstoff im Ausgangspräparat eingesetzt werden muss. Dies ist insbesondere dann relevant, wenn unerwünschte Nebenwirkungen eines Wirkstoffes, insbesondere im pharmazeutischen Bereich, verhindert werden sollen, oder wenn die Effektivität der Behandlung verbessert werden soll.

Mikroemulsionen sind ideale Formulierungen zur topischen, insbesondere dermalen Applikation von pharmazeutischen und kosmetischen Wirkstoffen. Sie verbessern die Bioverfügbarkeit der Wirkstoffe gegenüber herkömmlichen, aus dem Stand der Technik hinreichend bekannten Salben, Cremen, Lotionen und Gelen.

Mikroemulsionen weisen eine oder mehrere der folgenden Eigenschaften auf:
- Sie werden spontan gebildet, wenn ihre Komponenten miteinander in Kontakt gebracht werden;
- Sie sind praktisch nicht opaque, sondern transparent oder opaleszent, wenn sie unter einem optischen Mikroskop betrachtet werden; sie sind in ihrem nicht-gestörten Zustand optisch isotrop, obwohl sich bei einer Beobachtung beispielsweise unter Anwendung einer Röntgenstrahltechnik eine anisotrope Struktur feststellen lässt;
- Sie enthalten eine disperse oder stückige (Tröpfchen-) Phase, deren Teilchen eine Grösse von weniger als 200 nm haben, wovon ihre optische Transparenz herrührt.

Im Stand der Technik sind zur topischen Applikation, insbesondere zur dermalen Applikation im pharmazeutischen Bereich, vor allem Systeme gezeigt, bei denen die Mikroemulsion in Form eines wässerigen. Sprays aufgetragen wird. So zeigt beispielsweise WO 97/21428 einen Cortisolspray basierend auf einer Mikroemulsion mit Wasser als Trägerflüssigkeit.

Aufgrund ihrer wässerigen Beschaffenheit weisen die aus dem Stand der Technik bekannten Sprays aber eine schlechte Oberflächenhaftung, insbesondere auf der Haut, auf. Besonders nachteilig ist dabei, dass die applizierte Mikroemulsion von der Oberfläche, insbesondere von der Haut, abfliessen kann. Eine reproduzierbare Applikation, respektive Dosierung wird dabei erschwert oder verunmöglicht.

Liposomen sind kugelförmige Gebilde mit einem Durchmesser von 20 nm bis zu einigen µm. Sie bestehen aus mindestens einer Doppelschicht und umschliessen einen bestimmten wässrigen Raum. Abhängig von der Anzahl der das wässrige Innenvolumen umschliessenden Doppelschichten spricht man von unilamellaren (eine Doppelschicht), oligolamellaren (einige wenige Doppelschichten) sowie multilamellaren (viele Doppelschichten) Liposomen.

Im Gegensatz zu unilamellaren Liposomen, bei denen ein ihrer Grösse entsprechendes, wässriges Innenvolumen eingeschlossen ist, werden bei oligolamellaren bzw. multilamellaren Liposomen entsprechend der Anzahl an Doppelschichten mehrere voneinander getrennte wässrige Innenvolumen eingeschlossen.

Aufgrund ihrer physikochemischen Eigenschaften und ihrer Struktur können Liposomen als Träger für Wirkstoffe verwendet werden. Diese Wirkstoffe werden als Ergebnis ihrer hydrophilen und/oder lipophilen Eigenschaften entweder in den wässrigen Innenraum oder in die lipophilen Doppelschichten eingebracht oder an diese gebunden.

Pharmakodynamisch und/oder biologisch aktive Bilayerbildner bewirken, dass das Liposom selbst zum Arzneimittel wird. Je nach Applikationsart können die gebildeten, liposomalen Arzneimittel in entsprechende galenische Darreichungsformen überführt werden.

Liposomale Arzneimittel lassen sich zur gezielten Medikation für therapeutische und/oder diagnostische Zwecke sowie als Depotarzneiformen verwenden.

Ferner kann die Überführung des Arzneistoffes in ein liposomales Arzneimittel zur Verbesserung der Stabilität des Arzneistoffes und der resultierenden galenischen Darreichungsform führen.

Verfahren zur Herstellung von Liposomen und liposomalen Arzneimitteln sind herkömmlich bekannt (vgl. z.B. Liposomes: a practical approach, edited by R.R.C. New, Oxford University Press, 1990, auf welches ausdrücklich Bezug genommen wird).

Bei Liposomen tritt aufgrund ihrer wässerigen Beschaffenheit ebenfalls das Problem einer schlechte Oberflächenhaftung, insbesondere auf der Haut, auf. Besonders nachteilig ist dabei, dass die applizierte Lipsomensuspension von der Oberfläche, insbesondere von der Haut, abfliessen kann. Eine reproduzierbare Applikation, respektive Dosierung wird dabei erschwert oder verunmöglicht.

Aufgabe der vorliegenden Erfindung war es, die aus dem Stand der Technik bekannten Mängel zu vermeiden oder zu minimieren. Insbesondere soll eine Applikationsform zum Auftragen einer Mikroemulsion auf eine Oberfläche geschaffen werden, bei der ein gezieltes lokales Auftragen ermöglicht wird, welche eine reproduzierbare Applikation respektive Dosierung ermöglicht und welche insbesondere auch für topische, im besonderen für dermale Applikationen von Wirkstoffen im pharmazeutischen, respektive kosmetischen Bereich eingesetzt werden kann. Ausserdem soll die Bioverfügbarkeit verbessert werden.

Diese Aufgabe lässt sich überraschenderweise damit lösen, dass die Mikroemulsion in einer Schaumschicht auf das Substrat aufgebracht, insbesondere auf dem Substrat aufgeschäumt wird. Der Wirkstoff wird also in eine Mikroemulsion eingebracht und auf dem Substrat aufgeschäumt. Vor allem wird dabei die vorgängig mit Aufschäummittel und gegebenenfalls Schaumbildner versetzte Mikroemulsion aufgeschäumt, ohne, dass die Struktur der Teilchen der stückigen Phase, also die Partikel bzw. Nanopartikel der Mikroemulsion dabei in unerwünschter Weise verändert oder zerstört wird.

Gegenstand der Erfindung ist damit ein Verfahren zur Herstellung einer aufschäumbaren Dareichungsform gemäß den Anspruch 1, ein Verfahren zur Herstellung einer Mikroemulsion gemäß den Anspruch 3, ein Zusammensetzung gemäß den Anspruch 5, und deren Verwendung gemäß den Anspruch 22.

Wenn die Mikroemulsion in Form eines Schaums aus einem Behälter, insbesondere mit einer Sprühschaumvorrichtung, auf ein Substrat aufgebracht wird, lässt sich eine lokale Applikation an einer vorher definierten Stelle realisieren. Damit lässt sich nicht nur bezüglich Lokalität sondern auch hinsichtlich der aufgetragenen Menge eine reproduzierbare Applikation erreichen. Die erfindungsgemäss verwendeten Mikroemulsionen werden vorteilhaft vorgängig mit wenigstens einem Aufschäummittel und gegebenenfalls mit einem Schaumbildner versetzt, so dass sie selbstaufschäumend sind. Selbstaufschäumend im Sinne der Erfindung heisst, dass die Mikroemulsion erst beim Auftreffen auf der Oberfläche eines Substrates aufschäumt. Bei der anschliessenden spontanen Brechung des Schaums bildet sich ein hauchdünner Film. Während und nach dieser Filmbildung kann der in der Mikroemulsion enthaltene Wirkstoff über einen längeren Zeitraum kontinuierlich ins Substrat diffundieren. Besonders vorteilhaft ist dies, wenn die Mikroemulsion im pharmazeutischen oder kosmetischen Bereich topisch, insbesondere dermal appliziert wird. Gleichzeitig wird durch diesen Film die Transpiration der betroffenen Hautstelle reduziert. Als Folge davon nimmt die Hydratation der Haut zu, was die Wirkstoffaufnahme zusätzlich verbessern kann. Durch das erfindungsgemässe Verfahren wird zudem insbesondere eine schonende druckarme Applikation ermöglicht. Dies kann insbesondere bei der Behandlung von offenen Wunden oder Verbrennungen, beispielsweise mit Dexpanthenol, besonders vorteilhaft sein. Auch das Aufbringen von kosmetischen Wirkstoffen, wie beispielsweise von Q10 wird durch die Erfindung wesentlich verbessert.

Die mit Mikroemulsionen erreichbare hohe Bioverfügbarkeit der Wirkstoffe beruht darauf, dass der Wirkstoff in den Strukturen der Mikroemulsion gelöst vorliegt und damit in kleinsten Einheiten zur Aufnahme angeboten wird. Es ist deshalb erfindungswesentlich, dass die Struktur der Teilchen der stückigen Phase der Mikroemulsion durch das Aufschäumen nicht unerwünscht verändert oder zerstört wird. Dies lässt sich dadurch gewährleisten, dass Aufschäummittel und gegebenenfalls Schaumbildner im wesentlichen nicht in die Teilchen der stückigen Phase diffundieren. Bei der Auswahl von Aufschäummittel und Schaumbildner ist deshalb darauf zu achten, dass diese vorwiegend, vorzugsweise ausschliesslich in der wässerigen Phase der Mikroemulsion gelöst werden. Beim Aufschäumprozess wird so vor allem die wässerige Phase aufgeschäumt, während die Teilchen der stückigen Phase intakt bleiben.

Unter dem Begriff "im wesentlichen nicht in die Teilchen der stückigen Phase diffundieren" wird deshalb verstanden, dass die Teilchen der stückigen Phase nicht durch eindiffundiertes Aufschäummittel bei der Expansion des Aufschäummittels ebenfalls so expandieren, dass sie unerwünscht verändert oder zerstört werden. Bei der unerwünschten Veränderung oder Zerstörung der Teilchen der stückigen Phase bildet sich in der Regel eine Emulsion, d.h. die ursprünglichen Teilchen der stückigen Phase fusionieren zu wesentlich grösseren Teilchen, was unerwünscht ist. Dies lässt sich erfindungsgemäss mittels dynamischer Laserlicht-Streuung beispielsweise mit einem Nicomp 380 Particle Sizer überprüfen. Der dabei ermittelte mittlere Teilchendurchmesser der stückigen Phase soll auch nach dem Aufschäumen insbesondere maximal 200 nm, bevorzugterweise < 150 nm, noch bevorzugter < 100 nm betragen. Aufschäummittel wie Dymel 134a, Du Pont (1,1,1,2-Tetrafluorethan) diffundieren besonders leicht in die Teilchen der stückigen Phase, was vermieden werden soll.

Geeignete Aufschäummittel, die eine schonende Schaumbildung garantieren und die Teilchen der stückigen Phase nicht unerwünscht verändern oder zerstören sind flüssige Gase. Sie sollten bei Normaldruck (nach dem Ausbringen aus einem Schaumbehälter) bei einer Temperatur > -25°C, besonders vorteilhaft > 0°C von der flüssigen in die gasförmige Phase übergehen. Besonders bewährt haben sich Mischungen aus Isobutan und Isopentan. Art und Menge des Aufschäummittels sind jeweils auf die eingesetzte Mikroemulsion abzustimmen, so dass diese auch nach dem Aufschäumen strukturell intakt bleiben, d.h. dass ihre Teilchen der stückigen Phase nicht verändert oder zerstört werden.

Zum Auftragen einer Mikroemulsion in Form eines Schaums ist der Transfer der aufschäumbaren Mikroemulsion aus einem Behälter, insbesondere mit einer Sprühschaumvorrichtung, besonders vorteilhaft. Dazu muss die aufschäumbare Mikroemulsion in einem entsprechenden Behälter bereitgestellt werden.

Ein weiterer Gegenstand der Erfindung ist deshalb ein Verfahren zum Auftragen eines Wirkstoffs in Form einer selbstaufschäumenden Mikroemulsion auf ein Substrat.

Dieses Verfahren beinhaltet im wesentlichen folgende Schritte:
- Herstellen der Mikroemulsion enthaltend einen Wirkstoff.
- Zugabe von wenigstens einem Aufschäummittel und gegebenenfalls einem Schaumbildner, wobei Aufschäummittel und Schaumbildner im Hinblick auf die Zusammensetzung der Mikroemulsion so ausgewählt werden, dass sie im wesentlichen nicht in die Teilchen der stückigen Phase diffundieren.
- Abfüllung in einen Behälter, wobei der Behälter so als Schaumspender ausgebildet ist, dass die Mikroemulsion unter Ausbildung eines Schaums auf ein Substrat auftragbar ist.

Die Mikroemulsionen können dabei in bekannter Weise hergestellt werden, vorzugsweise durch Verdünnen eines Mikroemulsion-Prekonzentrates mit Wasser oder einer wässrigen Phase. Lipophile Wirkstoffe werden in das Mikroemulsion-Prekonzentrat eingearbeitet und durch Zugabe von Wasser oder einer wässerigen Phase in die Mikroemulsion übergeführt. Wasser lösliche Wirkstoffe werden vorab in Wasser oder einer wässrigen Phase gelöst. Durch Zugabe des Wirkstoff freien Mikroemulson-Prekonzentrats zu dieser Wirkstoff enthaltende Wasserphase bildet sich spontan die entsprechende Mikroemulsion. Es ist auch möglich, einen oder mehrere lipophile Wirkstoffe in das Mikroemulsions-Prekonzentrat einzuarbeiten und zusätzlich einen oder mehrere wasserlösliche Wirkstoffe der Mikroemulsion bzw. der Wasserphase für die Mikroemulsion zuzugeben.

Als Alternative ist auch denkbar, dass ein Behälter ein Mikroemulsions-Prekonzentrat und eine wässerige Phase mit wenigstens einem Aufschäummittel in voneinander getrennten Kompartimenten enthält und eine Vermischung der beiden Phasen erst unmittelbar vor Applikation erfolgt. Das Aufschäummittel wird dabei erfindungsgemäss vorteilhaft nur in der wässerigen Phase vorgelegt.

Lipophile (ölige) Wirkstoffe, welche in das Prekonzentrat für die Mikroemulsion (vor Zugabe der wässrigen Phase) eingearbeitet sind, werden Teil der "stückigen Phase" der Mikroemulsion, das heisst, sie werden in die Nanopartikel der Mikroemulsion integriert. Lipophile Wirkstoffe, die auf diese Weise in Mikroemulsionen eingearbeitet sind, werden meist wesentlich besser resorbiert, als Wirkstoffe die z.B. in Emulsionen enthalten sind.

Völlig überraschend hat es sich erfindungsgemäss aber auch gezeigt, dass auch wasserlösliche Wirkstoffe, welche vorab in die zur Mikroemulsionsbildung benötigte Wasserphase eingearbeitet werden und daher im wesentlichen an in der Wasser Phase der Mikroemulsion gelöst und/oder an der Grenzfläche stückige Phase/Wasser Phase angelagert sind, besser resorbiert werden.

Besonders gut aufschäumbare und auftragbare Zusammensetzungen lassen sich erreichen, wenn ein Mikroemulsion-Prekonzentrat verwendet wird, dass enthält :
(a1) ein Phospholipid
(a2) ein natürliches oder ein synthetisches oder ein parti alsynthetisches Di- oder Triglycerid, *sowie optional* ein Wachs, einen Fettalkohol, Derviate dieser Stoffe sowie Mischungen dieser Stoffe
(b) eine oberflächenaktive Komponente enthaltend ein Tensid vom Polyoxyethylentyp

Diese Mikroemulsion-Prekonzentrate können hergestellt werden, indem man die einzelnen Bestandteile, gegebenenfalls unter Erwärmen und Zuhilfenahme eines Lösungsvermittlers wie Ethanol, innig miteinander vermischt.

Die entsprechenden Mikroemulsionen sind erhältlich durch Verdünnen dieser Mikroemulsion-Prekonzentrate mit Wasser oder anderen wässrigen Flüssigkeiten. Dabei entstehen durch die im Prekonzentrat enthaltenen Komponenten (a1), (a2) und (b) spontan die Teilchen der stückigen Phase, deren Teilchengrösse kleiner als 200 nm, vorzugsweise kleiner als 150 nm, besonders bevorzugt kleiner als 100 nm ist. Ein weiterer Eintrag von Energie, insbesodere der Einsatz von hohen Scherkräften wird dabei nicht benötigt. Je nach der Menge des vorhandenen Wassers handelt es sich um W/O-Mikroemulsionen, um bikontinuierliche Mikroemulsionen oder O/W-Mikroemulsionen. Besonders bevorzugt im Rahmen der vorliegenden Erfindung sind dabei O/W-Mikroemulsionen.

Das Einbringen der Wirkstoffe in diese Mikroemulsionen erfolgt in bekannter Weise. Lipophile Wirkstoffe werden in das Mikroemulsion-Prekonzentrat und Wasser lösliche Wirkstoffe können vorteilhaft vorab in zur Mikroemulsionbildung benötigte Wasserphase eingearbeitet.

Vor allem für Mikroemulsionen, welche aus obigen Prekonzentraten enthaltend wenigstens (a1) ein Phospholipid, (a2) ein natürliches oder ein synthetisches oder ein partialsynthetisches Di- oder Triglycerid sowie optional, ein Wachs, ein Fettalkohol, Derviate dieser Stoffe sowie Mischungen dieser Stoffe und (b) eine oberflächenaktive Komponente enthaltend ein Tensid vom Polyoxyethylentyp hergestellt sind, haben sich als Aufschäummittel Isobutan und Isopentan bewährt. Besonders bevorzugt sind dabei Mischungen bestehend aus Isobutan und Isopentan.

Zur Unterstützung der Schaumbildung können die erfindungsgemäss eingesetzten Mikroemulsionen im weiteren wenigstens einen Schaumbildner enthalten. Dieser muss ebenfalls so ausgewählt sein, dass er im wesentlichen nicht in die Teilchen der stückigen Phase diffundiert, sondern bevorzugt in der wässerigen Phase löslich ist. Besonders bewährt haben sich dabei Schaumbildner ausgewählt aus der Gruppe der Betaine, Sulfosuccinate oder Sarcosin-Derivate. Diese sind beispielsweise unter den Bezeichnungen Betain 810 (Capryl/Capramidopropyl Betaine), Crodasinic LS35 (Aqua and Sodium Lauroyl Sarcosinate) und Rewopol SB FA 30 ((Disodium Lauroyl Sarcosinate) bekannt und im Handel erhältlich. Besonders bevorzugt ist dabei Rewopol SB FA 30.

Vorzugsweise enthält das erfindungsgemäss eingesetzte Mikroemulsion-Prekonzentrat als Komponente (a1) ein Phospholipid, ein hydriertes oder teilhydriertes Phospholipid, ein Lysophospholipid, ein Ceramid oder Mischungen aus diesen Verbindungen 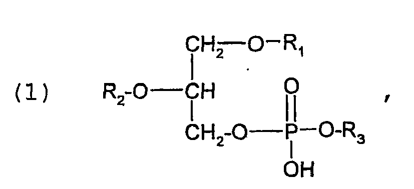 worin
R₁ C₁₀-C₂₀-Acyl;
R₂ Wasserstoff oder C₁₀-C₂₀-Acyl
R₃ Wasserstoff, 2-Trimethylamino-1-ethyl, 2-Amino-1-ethyl, nicht substituiertes oder durch eine oder mehrere Carboxy-, Hydroxy- oder Amino-Gruppen substituiertes C₁-C₅-Alkyl; die Inositol- oder die Glycerylgruppe;
bedeuten; oder Salze dieser Verbindungen.

C₁₀-C₂₀-Acyl ist vorzugsweise geradkettiges C₁₀-C₂₀-Alkanoyl mit einer geraden Anzahl an C-Atomen und geradkettiges C₁₀-C₂₀-Alkenoyl mit einer Doppelbindung und einer geraden Anzahl an C-Atomen.

Geradkettiges C₁₀-C₂₀-Alkanoyl mit einer geraden Anzahl an C-Atomen sind beispielsweise n-Dodecanoyl, n-Tetradecanoyl, n-Hexadecanoyl oder n-Octadecanoyl.

Geradkettiges C₁₀-C₂₀-Alkenoyl mit einer Doppelbindung und einer geraden Anzahl an C-Atomen sind beispielsweise 6-cis- oder-6-trans-, 9-cis- oder 9-trans-Dodecenoyl, -Tetradecenoyl, -Hexadecenoyl, -Octadecenoyl oder -Icosenoyl, insbesondere 9-cis-Octa-decenoyl (0leoyl), ferner 9,12-cis-Octadecadienoyl oder 9,12,15-cis-octadecatrienoyl.

Ein Phospholipid der Formel (1), worin R₃ 2-Trimethylamino-1-ethyl bedeutet, wird mit dem Trivialnamen Lecithin und ein Phospholipid der Formel (1), worin R₃ 2-Amino-1-ethyl bedeutet, mit dem Trivialnamen Kephalin bezeichnet. Geeignet sind beispielsweise natürlich vorkommendes Kephalin oder Lecithin, z.B. Kephalin oder Lecithin aus Sojabohnen oder Hühnerei mit verschiedenen oder identischen Acylgruppen oder Mischungen davon.

Das Phospholipid der Formel (1) kann aber auch synthetischen Ursprungs sein. Unter dem Begriff synthetisches Phospholipid definiert man Phospholipide, welche bezüglich R₁ und R₂ eine einheitliche Zusammensetzung haben. Solche synthetischen Phospholipide sind vorzugsweise die weiter oben definierten Lecithine und Kephaline, deren Acylgruppen R₁ und R₂ eine definierte Struktur haben und von einer definierten Fettsäure mit einem Reinheitsgrad höher als ca. 95% abgeleitet sind. R₁ und R₂ können gleich oder verschieden und ungesättigt oder gesättigt sein. Bevorzugt ist R₁ gesättigt, z.B. n-Hexadecanoyl, und R₂ ungesättigt, z.B. 9-cis-Octadecenoyl (Oleoyl).

Der Begriff "natürlich vorkommendes" Phospholipid definiert Phospholipide, welche bezüglich R₁ und R₂ keine einheitliche Zusammensetzung haben. Solche natürlichen Phospholipide sind ebenfalls Lecithine und Kephaline, deren Acylgruppen R₁ und R₂ von natürlich vorkommenden Fettsäuregemischen abgeleitet sind.

Die Forderung "im wesentlichen reines" Phospholipid der Formel (1) definiert einen Reinheitsgrad von mehr als 90 Gew.-%, vorzugsweise mehr als 95 Gew.-% des Phospholipids der Formel (1), welcher anhand geeigneter Bestimmungsmethoden, z.B. papier- oder dünnschichtchromatographisch, mit HPLC oder enzymatischem Farbtest, nachweisbar ist.

In einem Phospholipid der Formel (1) ist R₃ mit der Bedeutung C₁-C₄-Alkyl beispielsweise Methyl oder Ethyl. Die Bedeutung Methyl ist bevorzugt.

R₃ mit den Bedeutungen durch eine oder mehrere Carboxy-, Hydroxy- oder Aminogruppen substituiertes C₁-C₅-Alkyl sind beispielsweise 2-Hydroxyethyl, 2,3-Dihydroxy-n-propyl, Carboxymethyl, 1- oder 2-Carboxyethyl, Dicarboxymethyl, 2-Carboxy-2-hydroxyethyl oder 3-Carboxy-2,3-dihydroxy-n-propyl, 3-Amino-3-carboxy-n-propyl oder 2-Amino-2-carboxy-n-propyl, vorzugsweise 2-Amino-2-carboxyethyl.

Phospholipide der Formel (1) mit diesen Gruppen können in Salzform, z.B. als Natrium- oder Kaliumsalz, vorliegen.

Phospholipide der Formel (1), worin R₃ die Inositol- oder die Glycerylgruppe bedeutet, sind unter den Bezeichnungen Phosphatidylinositol und Phosphatidylglycerol bekannt.

Für die Acylreste in den Phospholipiden der Formel (1) sind auch die in Klammern angegebenen Bezeichnungen gebräuchlich: 9-cis-Dodecenoyl (Lauroleoyl), 9-Cis-Tetradecenoyl (Myristoleoyl), 9-cis-Hexadecenoyl (Palmitoleoyl), 6-cis-Octadecenoyl (Petroseloyl), 6-trans-Octadecenoyl (Petroselaidoyl), 9-cis-Octadecenoyl (Oleoyl), 9-trans-Octadecenoyl (Elaidoyl), 9,12-cis-Octadecadienoyl (Linoleoyl), 9,12,15-cis-Octadecatrienoyl (Linolenoyl), 11-cis-Octadecenoyl (Vaccenoyl), 9-cis-Icosenoyl (Gadoleoyl), 5,8,11,14-cis-Eicosatetraenoyl (Arachidonoyl), n-Dodecanoyl, (Lauroyl), n-Tetradecanoyl (Myristoyl), n-Hexadecanoyl (Palmitoyl), n-Octadecanoyl (Stearoyl), n-Icosanoyl (Arachidoyl), n-Docosanoyl (Behenoyl), n-Tetracosanoyl (Lignoceroyl).

Ein Salz des Phospholipids der Formel (1) ist vorzugsweise pharmazeutisch annehmbar. Salze definieren sich durch die Existenz von salzbildenden Gruppen im Substituenten R₃ sowie durch die freie Hydroxygruppe am Phosphoratom. Möglich ist ebenfalls die Bildung von inneren Salzen. Bevorzugt sind Alkalimetallsalze, insbesondere Natriumsalze.

In einer besonders bevorzugten Ausführungsform verwendet man gereinigtes Lecithin aus Sojabohnen der Qualität LIPOID S 100 oder S 75 oder ein Lecithin definiert in der Monographie USP23/NF 18.

Die Komponente (a1) wird vorzugsweise in einer Konzentration von ca. 0,1 bis 30 Gew.-%, bezogen auf das Gesamtgewicht der Komponenten (a1), (a2) und (b), eingesetzt.

Vorzugsweise enthält das erfindungsgemäss eingesetzte Mikroemulsion-Prekonzentrat als Komponente (a2) ein natürliches oder ein synthetisches oder ein partialsynthetisches Di- oder Triglycerid, optional ein Wachs, ein Fettalkohol, Derviate dieser Stoffe sowie Mischungen dieser Stoffe.

Bevorzugt sind mittelkettige Fettsäuretriglyceride vom Capryl-/Caprinsäure-Triglyceride Typ. Dazu gehören insbesondere die unter den Warenbezeichnungen MIGLYOL, MYRITOL, CAPTEX, CAP-MUL MCT, NEOBEE M5 und MAZOL 1400 bekannten und im Handel erhältlichen Zusammensetzungen (Fiedler, Lexikon der Hilfsstoffe, 3. Auflage, Seiten 808 bis 809 und 834, 1989).

Besonders bevorzugt ist dabei MIGLYOL 812, ein fraktioniertes Kokosnussöl, das Triglyceride von Caprylsäure und Caprinsäure enthält.

Die Komponente (a2) ist im erfindungsgemäss eingesetzten Mikroemulsion-Prekonzentrat in einer Konzentration von 0,1 bis 80 Gew.-%, bevorzugt in einer Konzentration von 10 bis 50 Gew.-%, bezogen auf das Gesamtgewicht der Komponenten (a1), (a2) und (b) vorhanden.

Bei Komponente (b) des erfindungsgemäss eingesetzten Mikroemulsion-Prekonzentrats, der oberflächenaktiven Komponente, enthaltend ein Tensid vom Polyoxyethylen-Typ, kann es sich um ein hydrophiles und/oder um ein lipophiles Tensid oder um Gemische solcher Tenside handeln. Nähere Angaben zu den.nachfolgend aufgeführten und im Handel erhältlichen Produkten finden sich in "Fiedler, Lexikon der Hilfsstoffe, 3. Auflage, 1989".

Beispiele für solche Tenside sind:
- Polyoxyethylenglykolierte natürliche oder hydrierte Pflanzenöle. Besonders geeignet sind CREMOPHOR, insbesondere CREMOPHOR RH 40, CREMOPHOR RH 60 und CREMOPHOR EL. Ferner NIKKOL, insbesondere NIKKOL HCO-60.
- Polyoxyethylensorbitanfettsäureester, wie sie beispielsweise unter der Bezeichnung TWEEN erhältlich sind, insbesondere TWEEN 80.
- Polyoxyethylenfettsäureester, beispielsweise MYRJ, insbesondere MYRJ 52 oder CETIOL HE.
- Copolymerisate von Polyoxyethylen und Polyoxypropylen wie PLURONIC, insbesondere PLURONIC F68, und EMKALYX
- Blockcopolymerisate von Polyoxyethylen und Polyoxypropylen, beispielsweise POLOXAMER, insbesondere POLOXAMER 188.
- Polyethoxylierte Vitamin E Derivate, insbesondere VITAMIN E TPGS (d-Alpha Tocoperyl Polyethylene Glycol 1000 Succinate, Eastman).
- Polyethoxylierte Hydroxyfettsäureester, insbesondere SOLUTOL HS 15 (Polyoxyethylen-660-Hydroxystearat, BASF).
- Umesterungsprodukte von natürlichen Pflanzenölglyceriden und Polyethylenpolyolen. Dazu gehören LABRAFIL, insbesondere LABRAFIL M 1944 CS und LABRAFIL M 2130 CS.
- Ethylenoxidaddukte von Sterolen und Derivaten davon, beispielweise GENEROL, insbesondere GENEROL 122 E5, 122 E10 und 122 E25.

Die erfindungsgemäss eingesetzten Mikroemulsion-Prekonzentrate umfassen sowohl Systeme, die ein einzelnes oberflächenaktives Mittel enthalten, als auch Systeme, die ein Gemisch von zwei oder mehreren oberflächenaktiven Mitteln enthalten, z.B. TWEEN 80 + CREMOPHOR RH 40, TWEEN 80 + CREMOPHOR RH 40 + VITAMIN E TPGS etc.

Vorzugsweise verwendet man eine oberflächenaktive Komponente, welche einen Polyoxyethylensorbitanfettsäureester, ein polyoxyethylenglykoliertes natürliches oder hydriertes Pflanzenöl oder Mischungen davon enthält.

Bevorzugterweise ist die oberflächenaktive Komponente (b) in den erfindungsgemäss verwendeten Mikroemulsion-Prekonzentraten in einer Menge von 20 bis 80 Gewichtsprozent bezogen auf das Gesamtgewicht des Prekonzentrates vorhanden.

Die erfindungsgemäss eingesetzten Mikroemulsion-Prekonzentrate und Mikroemulsionen können auch noch weitere Stoffe enthalten, wie z.B. Antioxidantien, Riech- und/oder Geschmacksstoffe, Farbstoffe, Konservierungsmittel, Hautpenetrationsverbesserer etc.

Die mit dem beschriebenen Verfahren hergestellte Mikroemulsionen eignen sich als Vehikel für Wirkstoffe. Diese können namentlich pharmazeutische, kosmetische oder biotechnologische Wirkstoffe oder generell Stoffe sein, die für einen bestimmten technischen Zweck eingesetzt werden.

Ein Beispiel eines pharmazeutischen Wirkstoffes, welcher mit den aufschäumbaren Mikroemulsionen besonders vorteilhaft appliziert werden kann, ist Dexpanthenol. Erfindungsgemäss können grundsätzlich alle pharmazeutischen Wirkstoffe, welche topisch, insbesondere dermal, appliziert werden können, mit den aufschäumbaren Mikroemulsionen aufgetragen werden. Vorzugsweise werden Wirkstoffe aus folgenden therapeutischen Klassen verwendet: Antiallergika, Antirheumatika/Entzündungshemmer, Lokalanästhetika, Venenmittel/Vasoprotektiva, Wundheilmittel, Hämorrhoidenmittel, Dermokortikoide, Antiseptika, Antibiotika, Antimykotika, antivirale Mittel, Antiparasitaria, Wirkstoffe zur Behandlung von Akne, Ekzemen, Psoriasis und Rosazea, Antipruriginosa, Antihidrotika, Adstringentia, Anitfibrotika, Desinfizientia, Wirkstoffe zur Behandlung von Hyperpigmentierung, Warzen und Hühneraugen, Wirkstoffe für Haar und Nägel, Hautschutz- und Pflegemittel, UV-Strahlenschutzmittel und Vitamine. Wirkstoffe der oben erwähnten therapeutischen Klassen können dem Arzneimittel Kompendium der Schweiz 2001, Documed AG, CH-4010 Basel, Herausgeber: Jürg Morant und Hans Ruppanner, entnommen werden.

Selbstverständlich können, falls therapeutisch gewünscht, auch Mischungen dieser Stoffe verwendet werden.

Weitere Wirkstoffe im Sinne der vorliegenden Erfindung sind Wirkstoffe, die sich für eine transdermale Therapie eigenen wie z.B. Hormone, Scopolamin und Nicotin.

Kosmetische Wirkstoffe im Sinne der vorliegenden Erfindung sind beispielsweise Aminosäuren, Peptide, Proteine oder deren Hydrolysate, Coenzyme, Flavonoide, UV-Filter und Vitamine.

Beispiele biotechnologischer Wirkstoffe sind Peptide und Proteine, insbesondere rekombinante Proteine. Beispiele von natürlichen und rekombinanten Proteinen sind Wachstumsfaktoren wie Epidermal Growth Factor (EGF), Keratinocyte Growth Factor (KGF), Acidic und Basic Fibroblast Growth Factor (aFGF und bFGF), Insulin-Like Growth Factor-I und II (IGF-I und IGF-II), Nerve Growth Factor (NGF), Platelet-Derived Growth Factors (PDGFs), Stem Cell Factors und Transforming Growth Factors; Chemotaktische Faktoren wie Macrophage/ Monocyte Chemotactic and Activating Factor (MCAF); Colony-Stimulating Faktoren wie Granulocyte-Macrophage Colony-Stimulating Factor (GM-CSF) und Granulocyte-Colony-Stimulating Factor (G-CSF); Interferone und Interleukine; Zelladhäsions- und Extracelluläre Matrix Proteine wie Kollagene, Fibronektine, Integrine, Laminine, Merosine, Proteoglykane, RGD-Adhäsionspeptide, Tenascine, Thrombospondine und Vitronektine und Enzyme wie Collagenase, Dispase und Trypsin. Weitere Beispiele von Proteinen sind Insulin, Albumin und Transferrin.

Technische Wirkstoffe im Sinne der Erfindung können alle Stoffe sein, die zum Erreichen eines bestimmten technischen Zwecks in die Mikroemulsion eingearbeitet werden.

Beispiele hierfür sind Schmierstoffe oder Markerstoffe, insbesondere Pigmente.

Zur Applikation der neuen Zusammensetzung und zur Durchführung des Verfahrens gemäss der vorliegenden Erfindung eignen sich Behälter in der Form von Sprühschaumspendern.

Besonders geeignete Behälter im Sinne der Erfindung sind Aerosolspraydosen, insbesondere ein- und zweikammerige Aerosolspraydosen. Diese ermöglichen eine Lagerung der mit Aufschäummittel und gegebenenfalls Schaumbildner versetzten Mikroemulsion. Die zum Ausbringen der Mikroemulsion eingesetzten Treibmittel sind so zu wählen, dass sie die Teilchen der stückigen Phase nicht unerwünscht verändern oder zerstören. Geeignete Treibmittel sind Flüssiggase oder komprimierte Gase, insbesondere komprimierter Stickstoff.

Weitere besonders gut aufschäumbare und auftragbare Zusammensetzungen lassen sich erreichen, wenn ein Mikroemulsion-Prekonzentrat verwendet wird, dass enthält:
(a) eine Mischung bestehend aus einem mittelkettigen Triglycerid und einer Omega-9-Fettsäure und/oder einer Omega-6-Fettsäure
(b) eine oberflächenaktive Komponente enthaltend ein Tensid vom Polyoxyethylentyp

Das Mikroemulsion-Prekonzentrat wird mit Wasser oder einem wässerigen Medium gemischt, wobei spontan die Mikroemulsion gebildet wird.

Das Einbringen der Wirkstoffe in die Mikroemulsion erfolgt in bekannter Weise. Lipophile Wirkstoffe werden in das Mikroemulsion-Prekonzentrat und Wasser lösliche Wirkstoffe werden vorteilhaft vorab in zur Mikroemulsionbildung benötigte Wasserphase eingearbeitet.

Aufschäummittel und gegebenenfalls Schaumbildner werden so ausgewählt, dass sie Teilchen der stückigen Phase nicht unerwünscht verändern oder zerstören.

Die zur Herstellung dieser Mikroemulsionen verwendeten Mikroemulsion-Prekonzentrate können hergestellt werden, indem man die einzelnen Bestandteile, gegebenenfalls unter Erwärmen, innig miteinander vermischt und, wie vorstehend beschrieben, mit Wasser verdünnt.

Das Mikroemulsion-Prekonzentrat enthält als Komponente (a) Mischungen aus einem mittelkettigen Fettsäuretriglycerid, zweckmässigerweise einem Fettsäuretriglycerid, in welchem die Fettsäurereste 4 bis 18, vorzugsweise 6 bis 18 C-Atome aufweisen, und einer Omega-9- und/oder einer Omega-6-Fettsäure.

Bevorzugte mittelkettige Fettsäuretriglyceride sind wie vorstehend beschrieben.

Besonders bevorzugt ist dabei MIGLYOL 812, ein fraktioniertes Kokosnussöl, das Triglyceride von Caprylsäure und Caprinsäure enthält.

Geeignete Omega-9-Fettsäuren sind hauptsächlich solche mit 12-24, insbesondere 16-24, vorzugsweise 18-22 C-Atomen, beispielsweise Oelsäure und Eicosatriensäure. Besonders bevorzugt ist die Oelsäure.

Geeignete Omega-6-Fettsäuren sind hauptsächlich solche mit 12-24, insbesondere 16-24, vorzugsweise 18-22 C-Atomen, beispielsweise Linolsäure, gamma-Linolensäure, dihommo-gamma-Linolensäure und Arachidonsäure. Besonders bevorzugt ist die Linolsäure.

In einer besonders bevorzugten Ausführungsform verwendet man als Komponente (a) eine Mischung bestehend aus einem Capryl-/Caprinsäure-Triglycerid, Oelsäure und/oder Linolsäure.

Bevorzugterweise ist die Komponente (a) in den erfindungsgemäss verwendeten Mikroemulsion-Prekonzentraten in einer Menge von 20 bis 70 Gewichtsprozent bezogen auf das Gesamtgewicht des Prekonzentrates vorhanden.

Das Mengenverhältnis von Omega-9-Fettsäure und/oder Omega-6-Fettsäure zum mittelkettigen Triglycerid liegt bevorzugt im Bereich 1:1 bis 1:200, noch bevorzugter im Bereich 1:2 bis 1:20.

Bei Komponente (b) der Mikroemulsion-Prekonzentrate, der oberflächenaktiven Komponente enthaltend ein Tensid vom Polyoxyethylen-Typ, kann es sich um ein hydrophiles und/oder um ein lipophiles Tensid oder um Gemische solcher Tenside handeln, wie vorstehend beschrieben wurde.

Beispiele solcher Tenside sind vorstehend beschrieben.

Die erfindungsgemäss eingesetzten Mikroemulsion-Prekonzentrate umfassen sowohl Systeme, die ein einzelnes oberflächenaktives Mittel enthalten, als auch Systeme, die ein Gemisch von zwei oder mehreren oberflächenaktiven Mitteln enthalten, z.B. TWEEN 80 + CREMOPHOR RH 40, TWEEN 80 + CREMOPHOR RH 40 + VITAMIN E TPGS etc.

Die Mikroemulsion-Prekonzentrate und Mikroemulsionen können auch noch weitere Stoffe enthalten, wie z.B. Antioxidantien, Riech- und/oder Geschmacksstoffe, Farbstoffe, Konservierungsmittel, Hautpenetrationsverbesserer etc.

Die mit dem beschriebenen Verfahren hergestellte Mikroemulsionen bzw. Liposomensuspensionen eignen sich als Vehikel für Wirkstoffe, wie vorstehend beschrieben.

Nachfolgend wird die vorliegende Erfindung anhand von Ausführungsbeispielen näher erläutert.

Die Beispiele 1-3 zeigen die Herstellung von Mikroemulsion-Prekonzentraten, Mikroemulsionen und selbstaufschäumenden Mikroemulsionen.

Das Beispiel 4 (nicht gemäß der Erfindung) zeigt die Herstellung einer selbstaufschäumenden Liposomensuspension.

### Beispiel 1: Herstellung von Mikroemulsion-Prekonzentraten

### Beispiel 1.1: Vitamin E Acetat 2% Mikroemulsion-Prekonzentrat

| | |
|---|---|
| Lecithin | 11.00% |
| Miglyol 812 | 48.00% |
| Tween 80 | 34.00% |
| Propylenglykol | 5.00% |
| Vitamin E Acetat | 2.00% |

Herstellung: Lecithin wird in Miglyol 812 gelöst. Zur erhaltenen Lösung fügt man Polysorbat 80, Propylenglykol und Vitamin E Acetat hinzu und rührt, bis man eine homogene, klare Flüssigkeit erhält.

### Beispiel 1.2: Coenzym Q10 1% Mikroemulsion-Prekonzentrat

| | |
|---|---|
| Lecithin | 11.00% |
| Miglyol 812 | 49.00% |
| Tween 80 | 34.00% |
| Propylenglykol | 5.00% |
| Coenzyme Q10 | 1.00% |

Herstellung: Lecithin wird in Miglyol 812 gelöst. Zur erhaltenen Lösung fügt man Polysorbat 80, Propylenglykol und Coenzym Q10 hinzu und rührt, gegebenenfalls unter leichtem Erwärmen, bis man eine homogene, klare Flüssigkeit erhält.

### Beispiel 2: Herstellung von Mikroemulsionen

Die in den Beispielen 1.1 und 1.2 beschriebenen Mikroemulsion-Prekonzentrate dienen nachfolgend als Basis zur Herstellung von Mikroemulsionen.

### Beispiel 2.1: Vitamin E Acetat 0.1% Mikroemulsion

| | |
|---|---|
| Mikroemulsion-Prekonzentrat, Beispiel 1.1 | 5.00 % |
| 10 mM Phosphatpuffer, pH 6 | ad 100,00 % |

Herstellung: Die Wasserphase wird unter Rühren (z.B. Magnetrührerwerk) bei 50°C vorgelegt. Das flüssige Mikroemulsion-Prekonzentrat, Beispiel 1.1, wird unter Rühren (z.B. mit einem Magnetrührwerk) der Wasserphase zugegeben.

### Beispiel 2.2: Coenzym Q10 0.05% Mikroemulsion

| | |
|---|---|
| Mikroemulsion-Prekonzentrat, Beispiel 1.2 | 5.00 % |
| 10 mM Phosphatpuffer, pH 6 | ad 100,00 % |

Herstellung: Die Wasserphase wird unter Rühren (z.B. Magnetrührerwerk) bei 50°C vorgelegt. Das flüssige Mikroemulsion-Prekonzentrat, Beispiel 1.2, wird unter Rühren (z.B. mit einem Magnetrührwerk) der Wasserphase zugegeben.

### Beispiel 3: Herstellung von selbstaufschäumenden Mikroemulsionen

Die in den Beispielen 2.1 und 2.2 beschriebenen Mikroemulsionen dienen nachfolgend als Basis zur Herstellung von selbstaufschäumenden Mikroemulsionen.

### Beispiel 3.1

### Selbstaufschäumende Vitamin E Acetat 0.1% O/W Mikroemulsion

| | |
|---|---|
| Dexpanthenol 5% W/O Mikroemulsion, Beispiel 2.1 | 96.0% |
| Rewopol SB FA 30 | 4.0% |
| Isobutan/Isopentan | q.s. |
| Komprimierter Stickstoff | q.s. |

Herstellung: Die Vitamin E Acetat 0.1% O/W Mikroemulsion, Beispiel 2.1, wird mit Rewopol SB FA 30 (Schaumbildner) versetzt, in Einkammer-Aerosoldosen abgefüllt und mit Isobutan/Isopentan (Aufschäummittel) sowie komprimiertem Stickstoff (Treibmittel) versetzt.

Aus nachfolgender Tabelle ist ersichtlich, dass die Teilchengrösse obiger Mikroemulsion vor und nach dem Aufschäumen vergleichbar ist.

**Tabelle 1:**

| Selbstaufschäumende Vitamin E Acetat 0.1% O/W Mikroemulsion: Teilchengrösse der nicht aufgeschäumten und aufgeschäumten O/W Mikroemulsion | |
|---|---|
| **Präparat** | **Teilchendurchmesser¹ [nm]** |
| Nicht aufgeschäumte O/W Mikroemulsion (enthält kein Aufschäummittel) | 28.3 ± 13.6 |
| Aufgeschäumte und wieder verflüssigte O/W Mikroemulsion | 32.2 ± 16.3 |

| | |
|---|---|
| ¹ Die Teilchendurchmesser und die Teilchengrössenverteilungen wurden mittels dynamischer Laserlicht-Streuung gemessen (Gerät: Nicomp 380 Submicron Particle Sizer, Auswertung: Volume Weighting). | |

### Beispiel 3.2

### Selbstaufschäumende Coenzym Q10 0.05% O/W Mikroemulsion

| | |
|---|---|
| Coenzym Q10 0.05% O/W Mikroemulsion, Beispiel 2.2 | 93.0% |
| Rewopol SB FA 30 | 4.0% |
| Isobutan/Isopentan | q.s. |

Die Coenzym Q10 0.05% O/W Mikroemulsion, Beispiel 2.2, wird mit Rewopol SB FA 30 (Schaumbildner) und Isobutan/Isopentan (Aufschäummittel) versetzt und in Zweikammer-Aerosoldosen, deren Aussenkammern mit dem Treibmittel n-Butan vorbegast wurden, abgefüllt.

Aus nachfolgender Tabelle ist ersichtlich, dass die Teilchengrösse obiger Mikroemulsion vor und nach dem Aufschäumen vergleichbar ist.

**Tabelle 2:**

| Selbstaufschäumende Coenzym Q10 0.05% O/W Mikroemulsion: Teilchengrösse der nicht aufgeschäumten und aufgeschäumten O/W Mikroemulsion | |
|---|---|
| **Präparat** | **Teilchendurchmesser² [nm]** |
| Nicht aufgeschäumte O/W Mikroemulsion (enthält kein Aufschäummittel) | 36.6 ± 17.8 |
| Aufgeschäumte und wieder verflüssigte O/W Mikroemulsion | 41.2 ± 21.1 |

| | |
|---|---|
| ² Die Teilchendurchmesser und die Teilchengrössenverteilung wurde mittels dynamischer Laserlicht-Streuung gemessen (Gerät: Nicomp 380 Submicron Particle Sizer, Auswertung: Volume Weighting). | |

### Beispiel 4 (nicht gemäß der Erfindung): Selbstaufschäumende Liposomensuspension

| | |
|---|---|
| Liposomensuspension | 99.5% |
| Rewopol SB FA 30 | 0.5% |
| Isobutan/Isopentan | q.s. |
| Komprimierter Stickstoff | q.s. |

Herstellung: Die Liposomensuspension, hergestellt nach gängigen Methoden (Liposomes: a practical approach, edited by R.R.C. New, Oxford University Press, 1990), wird mit Rewopol SB FA 30 (Schaumbildner) versetzt, in Einkammer-Aerosoldosen abgefüllt und mit Isobutan/Isopentan (Aufschäummittel) sowie komprimiertem Stickstoff (Treibmittel) versetzt.

Aus nachfolgender Tabelle ist ersichtlich, dass der Liposomendurchmesser obiger Liposomensuspension vor und nach dem Aufschäumen vergleichbar ist.

**Tabelle 3**

| Selbstaufschäumende Liposomensuspension: Liposomendurchmesser der nicht aufgeschäumten und aufgeschäumten Liposomen | |
|---|---|
| **Präparat** | **Liposomendurchmesser³ [nm]** |
| Nicht aufgeschäumte Liposomen (enthält kein Aufschäummittel) | 85.6 ± 41.9 |
| Aufgeschäumte und wieder verflüssigte Liposomen | 89.8 ± 47.8 |

| | |
|---|---|
| ³ Die Liposomendurchmesser und die Liposomengrössenverteilungen wurden mittels dynamischer Laserlicht-Streuung gemessen (Gerät: Nicomp 370 Submicron Particle Sizer, Auswertung: Volume Weighting). | |

## Patentansprüche

1. Verfahren zur Herstellung einer aufschäumbaren Darreichungsform, enthaltend die Schritte:
i) Bereitstellen einer Mikroemulsion enthaltend einen Wirkstoff, wobei die Mikroemulsion aus einem Mikroemulsions-Prekonzentrat hergestellt ist, enthaltend entweder
(a) eine Mischung bestehend aus einem Triglycerid mit Fettsäureresten mit 4-18 C-Atomen, und einer Omega-9-Fettsäure und/oder einer Omega-6-Fettsäure
(b) eine oberflächenaktive Komponente enthaltend ein Tensid vom Polyoxyethylentyp;
oder
(a1) ein Phospholipid
(a2) ein natürliches oder ein synthetisches oder ein partialsynthetisches Di- oder Triglycerid, sowie optional ein Wachs, einen Fettalkohol, Derviate dieser Stoffe sowie Mischungen dieser Stoffe
(b) eine oberflächenaktive Komponente enthaltend ein Tensid vom Polyoxyethylentyp;
ii) Bereitstellen von wenigstens einem Aufschäummittel und gegebenenfalls einem Schaumbildner, wobei Schaumbildner und Aufschäummittel im Hinblick auf die Zusammensetzung der Mikroemulsion so ausgewählt werden, dass sie im wesentlichen nicht in die Teilchen der stückigen Phase diffundieren, und das Aufschäummittel einen Siedepunkt > -25°C, vorzugsweise > 0°C aufweist;
iii) Bereitstellen eines Behälters zur Aufnahme der Mikroemulsion und Aufschäummittel sowie gegebenenfalls Schaumbildner.

2. Verfahren nach Anspruch 1, **dadurch gekennzeichnet, dass** der Behälter so als Schaumspender ausgebildet ist, dass die Mikroemulsion unter Ausbildung eines Schaumes auf ein Substrat auftragbar ist.

3. Verfahren zum Herstellen einer Mikroemulsion, insbesondere zur Verwendung in einem Verfahren gemäss einem der Ansprüche 1 oder 2, enthaltend die Schritte:
i) Bereitstellen eines Mikroemulsion-Prekonzentrates, enthaltend entweder
(a) eine Mischung bestehend aus einem Triglycerid mit Fettsäureresten mit 4-18 C-Atomen, und einer Omega-9-Fettsäure und/oder einer Omega-6-Fettsäure
(b) eine oberflächenaktive Komponente enthaltend ein Tensid vom Polyoxyethylentyp;
oder
(a1) ein Phospholipid
(a2) ein natürliches oder ein synthetisches oder ein partialsynthetisches Di- oder Triglycerid, sowie optional ein Wachs, einen Fettalkohol, Derviate dieser Stoffe sowie Mischungen dieser Stoffe
(b) eine oberflächenaktive Komponente enthaltend ein Tensid vom Polyoxyethylentyp;
ii) Vermischen des Mikroemulsion-Prekonzentrates mit Wasser oder einem wässrigen Medium, wobei spontan die Mikroemulsion gebildet wird;
iii) Zugabe von wenigstens einem Aufschäummittel und gegebenenfalls einem Schaumbildner, wobei Aufschäummittel und Schaumbildner im Hinblick auf die Zusammensetzung der Mikroemulsion so ausgewählt werden, dass sie im wesentlichen nicht in die Teilchen der stückigen Phase diffundieren, und das Aufschäummittel einen Siedepunkt > -25°C, vorzugsweise > 0°C aufweist.

4. Verfahren nach Anspruch 3, **dadurch gekennzeichnet, dass** der Wirkstoff in das Mikroemulsion-Prekonzentrat und/oder in die Wasserphase der Mikroemulsion eingebracht wird.

5. Zusammensetzung zum Aufbringen eines Wirkstoffs auf ein Substrat, wobei der Wirkstoff in einer Mikroemulsion gelöst ist, die Mikroemulsion ein Aufschäummittel und optional Schaumbildner enthält, sodass sie auf einem Substrat aufschäumbar ist, und wobei Aufschäummittel und Schaumbildner so gewählt wird, dass die Teilchen der stückigen Phase, das heisst die Partikel der Mikroemulsion im wesentlichen unzerstört bleiben, sodass die Teilchen der stückigen Phase beim Zerfallen des Schaums auf dem Substrat anlagerbar sind, und das Aufschäummittel einen Siedepunkt > -25°C, vorzugsweise > 0°C aufweist, wobei die Mikroemulsion aus einem Mikroemulsions-Prekonzentrat hergestellt ist, enthaltend entweder
(a) eine Mischung bestehend aus einem Triglycerid mit Fettsäureresten mit 4-18 C-Atomen, und einer Omega-9-Fettsäure und/oder einer Omega-6-Fettsäure
(b) eine oberflächenaktive Komponente enthaltend ein Tensid vom Polyoxyethylentyp;
oder
(a1) ein Phospholipid
(a2) ein natürliches oder ein synthetisches oder ein partialsynthetisches Di- oder Triglycerid, sowie optional ein Wachs, einen Fettalkohol, Derviate dieser Stoffe sowie Mischungen dieser Stoffe
(b) eine oberflächenaktive Komponente enthaltend ein Tensid vom Polyoxyethylentyp.

6. Zusammensetzung nach Anspruch 5, **dadurch gekennzeichnet, dass** das Aufschäummittel Isobutan, Isopentan oder Mischungen davon enthält.

7. Zusammensetzung nach einem der Ansprüche 5 bis 6, **dadurch gekennzeichnet, dass** der Schaumbildner ausgewählt ist aus der Gruppe der Betaine, Sulfosuccinate oder Sarcosin-Derivate.

8. Zusammensetzung nach einem der Ansprüche 5 bis 7, **dadurch gekennzeichnet, dass** sie eine O/W-Mikroemulsion mit einer durchschnittlichen Teilchengrösse unter 200 nm, bevorzugt unter 150 nm, besonders bevorzugt unter 100 nm ist.

9. Zusammensetzung nach einem der Ansprüche 5 bis 8, **dadurch gekennzeichnet, dass** das Triglycerid Fettsäurereste mit 6-18 C-Atomen aufweist.

10. Zusammensetzung nach einem der Ansprüche 5 bis 9, **dadurch gekennzeichnet, dass** die Omega-9-Fettsäure und/oder die Omega-6-Fettsäure 12-24, insbesondere 16-24, vorzugsweise 18-22 C-Atome aufweist.

11. Zusammensetzung nach einem der Ansprüche 5 bis 10 **dadurch gekennzeichnet, dass** sie als Komponente (a) eine Mischung aus einem Capryl-/Caprinsäure-Triglycerid, Ölsäure und/oder Linolsäure enthält.

12. Zusammensetzung nach einem der Ansprüche 5 bis 8, enthaltend als Komponente (a1) ein Phospholipid, ein hydriertes oder teilhydriertes Phospholipid, ein Lysophospholipid, ein Ceramid oder Mischungen aus diesen Verbindungen 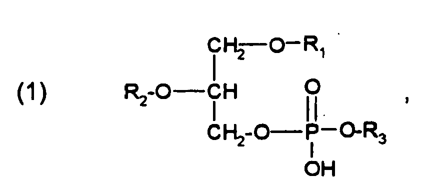 worin
R₁ C₁₀-C₂₀-Acyl;
R₂ Wasserstoff oder C₁₀-C₂₀-Acyl
R₃ Wasserstoff, 2-Trimethylamino-1-ethyl, 2-Amino-l-ethyl, nicht substituiertes oder durch eine oder mehrere Carboxy-, Hydroxy- oder Amino-Gruppen substituiertes C₁-C₅-Alkyl; die Inositol- oder die Glycerylgruppe;
bedeuten; oder Salze dieser Verbindungen.

13. Zusammensetzung nach einem der Ansprüche 5 bis 8 oder 12, enthaltend als Komponente (a2) ein Triglycerid mit Fettsäureresten mit 4-18 C-Atomen, vorzugsweise ein Capryl-/ Caprinsäure Trigycerid.

14. Zusammensetzung nach einem der Ansprüche 5 bis 13, **dadurch gekennzeichnet, dass** sie durch Vermischen eines Mikroemulsion-Prekonzentrates mit Wasser oder einem wässrigen Medium erhältlich ist.

15. Zusammensetzung nach einem der Ansprüche 5 bis 14 enthaltend einen Wirkstoff, insbesondere einen pharmazeutischen Wirkstoff, einen biotechnologischen Wirkstoff, einen kosmetischen Wirkstoff oder einen technischen Wirkstoff.

16. Zusammensetzung nach Anspruch 15, wobei der Wirkstoff lipophil ist und in den Teilchen der stückigen Phase einer Mikroemulsion gelöst ist.

17. Zusammensetzung nach Anspruch 15, wobei der Wirkstoff wasserlöslich ist und in der wässrigen Phase einer Mikroemulsion gelöst ist und/oder an der Grenzfläche stückige Phase/Wasserphase an- bzw. eingelagert ist.

18. Behälter enthaltend eine Zusammensetzung in Form einer Mikroemulsion nach einem der Ansprüche 5 bis 17.

19. Behälter enthaltend eine Zusammensetzung in Form eines Mikroemulsion-Prekonzentrates nach einem der Ansprüche 5 bis 17 und eine wässerige Phase mit wenigstens einem Aufschäummittel.

20. Behälter nach einem der Ansprüche 18 oder 19, **dadurch gekennzeichnet, dass** der Behälter ein Sprühschaumspender, insbesondere eine Aerosolspraydose, insbesondere eine ein- oder zweikammerige Aerosolspraydose, ist.

21. Behälter nach einem der Ansprüche 19 bis 20, **dadurch gekennzeichnet dass** der Behälter wenigstens ein Treibmittel, insbesondere ein Flüssiggas oder komprimiertes Gas, vorzugsweise komprimierter Stickstoff, enthält.

22. Verwendung einer Zusammensetzung nach einem der Ansprüche 5 bis 17 zur Herstellung einer, eine Mikroemulsion mit wenigstens einem pharmazeutischen Wirkstoff enthaltenden, schaumförmigen Darreichungsform auf einem Substrat, zur Behandlung von Krankheiten.

23. Verwendung nach Anspruch 22, wobei die Mikroemulsion in Form eines Schaums auf die Haut aufgetragen wird.

24. Verwendung nach Anspruch 22, wobei die Mikroemulsion wenigstens einen biotechnologischen Wirkstoff, insbesondere ein Protein oder ein Peptid, enthält.

25. Verwendung einer Zusammensetzung nach einem der Ansprüche 5 bis 17 zum Auftragen einer Mikroemulsion mit wenigstens einem kosmetischen Wirkstoff oder wenigstens einem technischen Wirkstoff in Form eines Schaums auf die Haut.

26. Verwendung eines ein- oder zweikammerigen Schaumspendebehälters, enthaltend eine Zusammensetzung nach einem der Ansprüche 5 bis 17, zur Herstellung einer aufschäumenden Mikroemulsion.

27. Verwendung nach einem der Ansprüche 22 bis 26, wobei der Wirkstoff Dexpanthenol ist.

## Claims

1. Method for the manufacture of a foamable composition, containing the steps:
i) Provision of a microemulsion containing an active ingredient, whereby the microemulsion is manufactured from a microemulsion pre-concentrate, containing either
(a) a mixture consisting of a triglyceride with fatty acid residues with 4-18 C atoms, and an Omega-9 fatty acid and/or an Omega-6 fatty acid
(b) a surface-active component containing a tenside of the polyoxyethylene type; or
(a1) a phospholipid
(a2) a natural or a synthetic or a partially synthetic di- or triglyceride, also optionally a wax, a fatty alcohol, derivatives of these substances as well as mixtures of these substances
(b) a surface-active component containing a tenside of the polyoxyethylene type;
ii) Provision of at least one frothing reagent and, if necessary, a foaming agent, whereby the foaming agent and frothing reagent are selected with due consideration of the composition of the microemulsion, such that essentially they do not diffuse in the particles of the solid phase, and the frothing reagent has a boiling point > -25°C, preferably >0°C;
iii) Provision of a container for the acceptance of the microemulsion and frothing reagent and, if necessary, the foaming agent.

2. Method in accordance with claim 1, **characterised in that** the container is formed as a foam dispenser, in such a way, that the microemulsion can be applied to a substrate as a foam is formed.

3. Method for the manufacture of a microemulsion, in partucular for use in a method according to any one of claims 1 or 2, containing the steps:
i) Provision of a microemulsion pre-concentrate, containing either
(a) a mixture consisting of a triglyceride with fatty acid residues with 4-18 C atoms, and an Omega-9 fatty acid and/or an Omega-6 fatty acid
(b) a surface-active component containing a tenside of the polyoxyethylene type;
or
(a1) a phospholipid
(a2) a natural or a synthetic or a partially synthetic di- or triglyceride, also optionally a wax, a fatty alcohol, derivatives of these substances as well as mixtures of these substances
(b) a surface-active component containing a tenside of the polyoxyethylene type;
(ii) Mixing of the microemulsion pre-concentrate with water or an aqueous medium, whereby the microemulsion is formed spontaneously;
(iii) Addition of at least one frothing reagent and, if necessary, a foaming agent, whereby the foaming agent and frothing reagent are selected with due consideration of the composition of the microemulsion, such that essentially they do not diffuse in the particles of the solid phase, and the frothing reagent has a boiling point > -25°C, preferably >0°C;

4. Method according to Claim 3, **characterised in that** the active ingredient is brought in in the microemulsion pre-concentrate and/or in the aqueous phase of the microemulsion.

5. Composition for the deposition of an active ingredient on a substrate, whereby the active ingredient is dissolved in a microemulsion, the microemulsion contains a frothing reagent and optional foaming agent, so that it can be frothed onto a substrate and whereby the frothing reagent and foaming agent are selected such that the particles in the solid phase, i.e. the particles of the microemulsion remain mostly undestroyed, so that the particles of the solid phase can be added to the substrate as the foam disintegrates, and the frothing reagent has a boiling point > - 25°C, preferably > 0°C, whereby the microemulsion is manufactured from a microemulsion pre-concentrate, containing either
(a) A mixture consisting of a triglyceride with fatty acid residues with 4-18 C atoms, and an Omega-9 fatty acid and/or an Omega-6 fatty acid
(b) a surface-active component containing a tenside of the polyoxyethylene type;
or
(a1) a phospholipid
(a2) a natural or a synthetic or a partially synthetic di- or triglyceride, also optionally a wax, a fatty alcohol, derivatives of these substances as well as mixtures of these substances
(b) a surface-active component containing a tenside of the polyoxyethylene type;

6. Composition according to claim 5, **characterised in that** the frothing reagent contains isobutane, isopentane or mixtures of these.

7. Composition according to any one of claims 5 or 6, **characterised in that** the foaming agent is selected from the group of the betains, sulfosuccinates or sarcosin derivatives.

8. Composition according to any one of claims 5 to 7, **characterised in that** it is an O/W microemulsion with an average particle size under 200 nm, preferably under 150 nm and in particular, preferably under 100 nm.

9. Composition according to any one of claims 5 to 8, **characterised in that** the triglyceride fatty acid residue has 6-18 C atoms.

10. Composition according to any one of claims 5 to 9, **characterised in that** the Omega-9 fatty acid and/or the Omega-6 fatty acid has 12-24, in particular 16-24, and preferably 18-22 C atoms.

11. Composition according to any one of claims 5 to 10, **characterised in that** it contains as component (a) a mixture of a caprylic/capric acid triglyceride, oleic acid and/or linoleic acid.

12. Composition according to any one of claims 5 to 8, containing as component (a1) a phospholipid, a hydrogenated or partially hydrogenated phospholipid, a lysophospholipid, a ceramide or mixtures of these compounds. 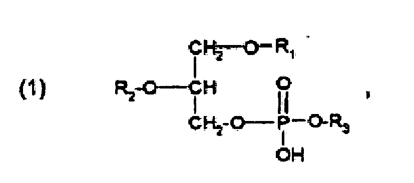 where
R₁ is C₁₀-C₂₀-acyl
R₂ is hydrogen or C₁₀-C₂₀-acyl
R₃ is hydrogen, 2-trimethylamino-1-ethyl, 2-amino-1-ethyl, unsubstituted C₁-C₅-alkyl or C₁-C₅-alkyl substituted by one or more carboxy, hydroxy or amino groups; the inositol or glyceryl groups; or salts of these compounds.

13. Composition according to any one of claims 5 to 8 or 12, containing as component (a2) a triglyceride with fatty acid residues with 4-18 C-atoms, preferably a caprylic acid/capric acid triglyceride.

14. Composition according to any one of claims 5 to 13, **characterised in that** it is obtainable through mixing a microemulsion pre-concentrate with water or an aqueous medium.

15. Composition according to any one of claims 5 to 14, containing an active substance, in particular a pharmaceutical substance, a biotechnological substance, a cosmetic substance or a technical substance.

16. Composition according to claim 15, whereby the active substance is lipophilic and is dissolved in the particles in the solid phase of a microemulsion.

17. Composition according to claim 15, whereby the active substance is soluble in water and is dissolved in the aqueous phase of a microemulsion and/or dispersed in or added to the boundary surface of the solid phase/water phase.

18. Container containing a composition in the form of a microemulsion according to any one of claims 5 to 17.

19. Container containing a composition in the form of a microemulsion pre-concentrate according to any one of claims 5 to 17 and an aqueous phase with at least one frothing reagent.

20. Container according to any one of claims 18 or 19, **characterised in that** the container is a spray foam dispenser, in particular an aerosol spray can, in particular a one or two-chamber aerosol spray can.

21. Container according to any one of claims 19 or 20, **characterised in that** the container contains at least one propellant, in particular a liquid gas or compressed gas, preferably compressed nitrogen.

22. Use of a composition according to any one of claims 5 to 17 for the manufacture of a foam-type composition on a substrate, containing a microemulsion with at least one pharmaceutical substance, for the treatment of illnesses.

23. Use according to claim 22, whereby the microemulsion is applied to the skin in the form of a foam.

24. Use according to claim 22, whereby the microemulsion contains at least one active biotechnological substance, in particular a protein or a peptide.

25. Use of a composition according to any one of claims 5 to 17 for the application on the skin of a microemulsion with a least one cosmetic substance or at least one technical substance in the form of a foam.

26. Use of a one or two-chamber foam dispensing container, containing a composition according to any one of claims 5 to 17, for the manufacture of a foaming microemulsion.

27. Use according to any one of claims 22 to 26, whereby the substance is Dexpanthenol.

## Revendications

1. Procédé pour préparer une forme galénique moussante, comprenant les étapes consistant à :
i) réaliser une microémulsion contenant un principe actif, la microémulsion étant préparée à partir d'un préconcentré de microémulsion, contenant soit
(a) un mélange constitué par un triglycéride avec des esters d'acides gras ayant 4 à 18 atomes de carbone et un acide gras oméga-9 et/ou un acide gras oméga-6,
(b) un composant tensioactif contenant un tensioactif du type polyoxyéthylène ; soit
(a1) un phospholipide,
(a2) un diglycéride ou un triglycéride naturel, de synthèse ou partiellement de synthèse ainsi que, facultativement, une cire, un alcool gras, un dérivé de ces substances ainsi qu'un mélange de ces substances,
(b) un composant tensioactif contenant un tensioactif du type polyoxyéthylène,
ii) réaliser au moins un agent moussant et le cas échéant un agent de formation de mousse, ce dernier et l'agent moussant étant choisis en fonction de la composition de la microémulsion pour qu'ils ne diffusent essentiellement pas dans les particules de la phase solide et l'agent moussant ayant un point d'ébullition supérieur à -25°C, de préférence supérieur à 0 °C,
iii) réaliser un contenant pour recueillir la microémulsion et l'agent moussant ainsi que le cas échéant, l'agent de formation de mousse.

2. Procédé selon la revendication 1, **caractérisé en ce que** le contenant est fabriqué sous forme de distributeur de mousse et **en ce que** la microémulsion peut être déposée sur un substrat lors de la formation d'une mousse.

3. Procédé pour préparer une microémulsion, notamment pour l'utiliser dans un procédé selon l'une des revendications 1 et 2, comprenant les étapes consistant à :
i) réaliser un préconcentré de microémulsion, contenant soit
(a) un mélange constitué par un triglycéride avec des esters d'acides gras ayant 4 à 18 atomes de carbone et un acide gras oméga-9 et/ou un acide gras oméga-6,
(b) un composant tensioactif contenant un tensioactif du type polyoxyéthylène ;
soit
(a1) un phospholipide,
(a2) un diglycéride ou un triglycéride naturel, de synthèse ou partiellement de synthèse ainsi que, facultativement, une cire, un alcool gras, un dérivé de ces substances ainsi qu'un mélange de ces substances,
(b) un composant tensioactif contenant un tensioactif du type polyoxyéthylène,
ii) mélanger le préconcentré de microémulsion à de l'eau ou à un milieu aqueux, ce qui forme spontanément la microémulsion ;
iii) ajouter au moins un agent moussant et le cas échéant un agent de formation de mousse, ce dernier et l'agent moussant étant choisis en fonction de la composition de la microémulsion pour qu'ils ne diffusent essentiellement pas dans les particules de la phase solide et l'agent moussant ayant un point d'ébullition supérieur à -25°C, de préférence supérieur à 0 °C.

4. Procédé selon la revendication 3, **caractérisé en ce que** le principe actif est incorporé dans le préconcentré de microémulsion et/ou dans la phase aqueuse de la microémulsion.

5. Composition pour déposer un principe actif sur un substrat, le principe actif étant dissous dans une microémulsion, la microémulsion contenant un agent moussant et un agent de formation de mousse facultatif de manière à ce qu'elle puisse mousser sur un substrat, et l'agent moussant et l'agent de formation de mousse étant choisis de manière à ce que les particules de la phase solide, c'est-à-dire les particules de la microémulsion, restent essentiellement intactes pour que les particules de la phase solide puissent être déposées sur le substrat lorsque la mousse se désagrège, et le moyen moussant ayant un point d'ébullition supérieur à -25°C, de préférence supérieur à 0 °C et la microémulsion étant préparée à partir d'un préconcentré de microémulsion, contenant soit
(a) un mélange constitué par un triglycéride avec des esters d'acides gras ayant 4 à 18 atomes de carbone et un acide gras oméga-9 et/ou un acide gras oméga-6,
(b) un composant tensioactif contenant un tensioactif du type polyoxyéthylène ; soit
(a1) un phospholipide,
(a2) un diglycéride ou un triglycéride naturel, de synthèse ou bien partiellement de synthèse ainsi que, facultativement, une cire, un alcool gras, un dérivé de ces substances ainsi qu'un mélange de ces substances,
(b) un composant tensioactif contenant un tensioactif du type polyoxyéthylène.

6. Composition selon la revendication 5, **caractérisée en ce que** l'agent moussant contient de l'isobutane, de l'isopentane ou des mélanges de ceux-ci.

7. Composition selon l'une des revendications 5 à 6, **caractérisée en ce que** l'agent de formation de mousse est choisi dans le groupe constitué par la bétaïne, le sulfosuccinate ou des dérivés de la sarcosine.

8. Composition selon l'une des revendications 5 à 7, **caractérisée en ce qu'**elle est une microémulsion H/E ayant une granulométrie moyenne inférieure à 200 nm, de préférence inférieure à 150 nm, et de manière particulièrement préférée inférieure à 100 nm.

9. Composition selon l'une des revendications 5 à 8, **caractérisée en ce que** le triglycéride présente des restes d'acides gras ayant 6 à 18 atomes de carbone.

10. Composition selon l'une des revendications 5 à 9, **caractérisée en ce que** l'acide gras oméga-9 et/ou l'acide gras oméga-6 contien(nen)t notamment 16 à 24, de préférence 18 à 22 atomes de carbone.

11. Composition selon l'une des revendications 5 à 10, **caractérisée en ce qu'**elle contient comme composant (a) un mélange constitué par un triglycéride d'acide caprylique/caprique, l'acide oléique et/ou l'acide linoléique.

12. Composition selon l'une des revendications 5 à 8, contenant comme composant (a1) un phospholipide, un phospholipide hydrogéné ou partiellement hydrogéné, un lysophospholipide, un céramide ou des mélanges de ces composés 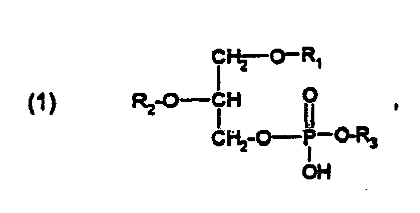 où
R₁ désigne acyle en C₁₀-C₂₀ ;
R₂ désigne hydrogène ou acyle en C₁₀-C₂₀ ;
R₃ désigne hydrogène, 2-triméthylamino-1-éthyle, 2-amino-1-éthyle, alkyle en C₁-C₅ non substitué ou substitué par un ou plusieurs groupes carboxy, hydroxy ou amino ; le groupe inositol ou le groupe glycéryle ; ou des sels de ces composés.

13. Composition selon l'une des revendications 5 à 8 et 12, contenant comme composant (a2) un triglycéride avec des restes d'acides gras ayant 4 à 18 atomes de carbone, de préférence un triglycéride d'acide caprylique/caprique.

14. Composition selon l'une des revendications 5 à 13, **caractérisée en ce qu'**elle peut être obtenue en mélangeant un préconcentré de microémulsion avec de l'eau ou avec un milieu aqueux.

15. Composition selon l'une des revendications 5 à 14, contenant un principe actif, notamment un principe actif pharmaceutique, un principe actif biotechnologique, un principe actif cosmétique ou un principe actif technique.

16. Composition selon la revendication 15, le principe actif étant lipophile et dissous dans les particules de la phase solide d'une microémulsion.

17. Composition selon la revendication 15, le principe actif étant hydrosoluble et dissous dans la phase aqueuse d'une microémulsion et/ou déposé au niveau de l'interface phase solide/phase aqueuse ou incorporé à celle-ci.

18. Contenant renfermant une composition sous la forme d'une microémulsion selon l'une des revendications 5 à 17.

19. Contenant renfermant une composition sous la forme d'un préconcentré de microémulsion selon l'une des revendications 5 à 17 et une phase aqueuse contenant au moins un agent moussant.

20. Contenant selon l'une des revendications 18 et 19, **caractérisé en ce qu'**il est un pulvérisateur de mousse, notamment une bombe aérosol, plus particulièrement une bombe aérosol à une ou deux chambres.

21. Contenant selon l'une des revendications 19 à 20, **caractérisé en ce qu'**il contient au moins un gaz propulseur, notamment un gaz liquide ou un gaz comprimé, de préférence de l'azote comprimé.

22. Utilisation d'une composition selon l'une des revendications 5 à 17 pour préparer une forme galénique sous forme de mousse contenant une microémulsion renfermant au moins un principe pharmaceutique sur un substrat pour le traitement de maladies.

23. Utilisation selon la revendication 22, la microémulsion étant appliquée sur la peau sous forme de mousse.

24. Utilisation selon la revendication 22, la microémulsion contenant au moins un principe biotechnologique, notamment une protéine ou un peptide.

25. Utilisation d'une composition selon l'une des revendications 5 à 17, pour l'application sur la peau d'une microémulsion ayant au moins un principe actif cosmétique ou au moins un principe actif technique, sous forme de mousse.

26. Utilisation d'un distributeur de mousse à une ou deux chambres contenant une composition selon l'une des revendications 5 à 17 pour préparer une microémulsion moussante.

27. Utilisation selon l'une des revendications 22 à 26, le principe actif étant le dexpanthénol.
